(19) Europäisches Patentamt
European Patent Office
Office européen des brevets

(11) **EP 4 120 278 A1**

(12) **EUROPEAN PATENT APPLICATION**

(43) Date of publication:
**18.01.2023 Bulletin 2023/03**

(21) Application number: **21186218.0**

(22) Date of filing: **16.07.2021**

(51) International Patent Classification (IPC):
**G16B 25/10** (2019.01)   **G16B 5/30** (2019.01)
**G16B 40/20** (2019.01)

(52) Cooperative Patent Classification (CPC):
**G16B 25/10; G16B 5/30; G16B 40/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Shift Bioscience Ltd.
Cambridge CB22 3AT (GB)**

(72) Inventor: **Swain, Brendan
Cambridge, CB22 3AT (GB)**

(74) Representative: **Korenberg, Alexander Tal
Kilburn & Strode LLP
Lacon London
84 Theobalds Road
London WC1X 8NL (GB)**

(54) **TEMPORAL PROPERTY PREDICTOR**

(57)    Computer-implemented method of obtaining a predictor for predicting a time-varying property based on gene transcription data are provided. The methods comprise receiving a data set comprising data samples obtained from respective cell samples having different values of the time-varying property, each data sample comprising a number of transcription levels, and a respective actual value of the time-varying property of the cell sample for each data sample, wherein each transcription level is a transcription level of an individual gene transcript or a pooled transcription level of gene transcripts of an individual gene; generating an embedded data set comprising for each data sample an embedded sample, wherein a number of dimensions of the embedded samples is less than the number of transcription levels; applying the embedded data set as an input to the predictor to produce a predicted value of the time-varying property for each embedded sample; and obtaining the predictor by adjusting prediction coefficients of the predictor to reduce an error measure of prediction error between respective predicted and actual values of the time-varying property. The time varying property may be age, for example biological age or progression of a disease, disorder or condition.

Figure 1

EP 4 120 278 A1

**Description**

TECHNICAL FIELD

This disclosure relates to computer implemented methods, and corresponding computer program products, computer readable media and systems, for obtaining a predictor for predicting time-varying properties from gene transcripts. In particular but not exclusively, the disclosure relates to predictors for biological or chronological age or progression of a disease, disorder or condition. Another non-exclusive aspect of the disclosure relates to estimating the contribution to the prediction of different genes or gene transcripts.

BACKGROUND

Many diseases have an aging component, e.g. Parkinson's disease, Alzheimer's disease and osteoarthritis. There is growing interest in identifying ways to induce cellular and tissue regeneration with novel therapeutics that can unlock the latent regeneration capabilities that are present in very young cells. In the last five years there have been many advances in the science in a field called partial epigenetic re-programming, which holds great promise.

The only comprehensive way previously known to induce cells to transform to a younger state was to create iPSCs (e.g. using Yamanaka factors). Unfortunately, cells undergoing this shift to pluripotency also change their identity, so the technique cannot be used for creating anti-aging therapeutics, nor to extend health span.

It is now known from studies of partial epigenetic reprogramming that the age-reversing component can be decoupled from the cell identity component, and efforts are now ongoing to translate this process to the clinic.

Aging clocks are an elegant way to understand how to drive the cellular rejuvenation process. The first aging clock was developed by Horvath *et al.* (see for example US20160222448A1 and US20190185938A1) and is based on methylation levels, hence being described as an epigenetic clock. Although they predict age highly accurately, epigenetic clocks have several limitations, including difficulty in making biological inferences and the current inability to validate or target individual sites for potential therapeutic benefit. Attention has therefore turned to transcriptomic clocks, which predict age based on RNA expression levels. Transcriptomic clocks have been described, for example, in US10325673B2 and by Holzscheck et al. (npj Aging Mech Dis 7, 15 (2021)). However, a significant feature of these transcriptomic clocks is that they operate on summarised transcription levels for corresponding gene pathways and therefore require knowledge of the gene pathways up front in order to make such clocks. The inventors have realised that this has a number of drawbacks, as explained below. There is therefore a need in the art for a clock (predictor of aging) which overcomes these limitations.

SUMMARY

Aspects of an invention are set out in the accompanying independent claims. Optional features of some embodiments are set out in the dependent claims.

The disclosure provides a computer-implemented method for obtaining a predictor for predicting a time-varying property based on gene transcription data (i.e. RNA expression levels). Aging clocks are an example of a predictor of a time varying property (age) but it will be appreciated that the disclosure is not limited to age as the time-varying property and is applicable to other time-varying properties.

The method comprises receiving a data set comprising data samples obtained from respective cell samples having different values of the time-varying property. The cell samples may be single cells or collections of cells over which transcription levels are pooled to form the data samples. The cell samples may be obtained from cell cultures *in vitro,* for example. Alternatively, the cell samples may be obtained from an individual, for example by taking a biopsy. The step of obtaining the cell samples typically does not form part of the method. Each data sample comprises a number of transcription levels. Each data sample further comprises a respective actual value of the time-varying property of the cell sample for each data sample. The time-varying property may be biological or chronological age, a progression or stage of a disease or condition, for example cancer or neurodegenerative conditions such as Alzheimer's disease or Parkinson's disease, and the like. It can therefore be seen that although the respective cell samples have different values of the time-varying property, the respective cell samples may all be taken at the same time, but represent, for example, different stages of progression of a disease or condition. The time-varying property may be of one or more organisms or subjects from which the cell samples have been obtained.

Each transcription level is a transcription level of an individual gene transcript or a pooled transcription level of gene transcripts of an individual gene. The transcription levels may thus be obtained from the corresponding transcription counts of individual genes or gene transcripts in the respective cell samples. For example, in some implementations, the transcription counts may be obtained using transcriptomic techniques such as RNA-Seq.

Because the method operates on individual gene transcripts or genes, any bias associated with the definition and selection of pathways may be avoided. Further, new genes that are involved in bringing about the time-varying property can, in some implementations, be discovered. Since knowledge of gene pathways or biological activity is not required, unlike prior approaches of the state of the art, transcription levels derived from transcription counts of gene transcripts in the cell samples can be used in the analysis without use of knowledge of gene pathways or biological activity.

The method comprises generating, from the individual transcription levels, an embedded data set comprising for each data sample an embedded sample. The number of dimensions of the embedded samples is less than the number of transcription levels, such that the embedding provides a dimensionality reduction. In some implementations, the number of dimensions of the embedded samples may be selected based on the respective prediction performance of embedded data sets having different respective numbers of dimensions. Advantageously, by reducing the number of dimensions, computational efficiency is enhanced and can help reduce the amount of variance that is driven by technical noise. This may be particularly advantageous in case of single cell samples, where technical noise can be large compared to biological signals.

In some implementations, the method may comprise applying a transformation to the data set to generate the embedded data set. The transformation may be obtained by operating on the data set, for example by operating on a covariance matrix of the data set. In some implementations, the transformation may be obtained without using knowledge of gene pathways.

In some implementations, the embedding may comprise a linear transformation of the transcription data set to generate the embedded data set and in some specific implementations, the embedded data set comprises a subset of the principal components of the transcription data set. In some implementations, a non-linear mapping may be used.

The method may comprise, in some implementations, applying an inverse mapping to the prediction coefficients to project the prediction coefficients onto the dimensions of the transcription data set. The inverse mapping maps from embedded cell samples to corresponding cell samples. In this way, a measure of contribution to predicting a value of the time-varying property can be derived for each gene or transcript. In some cases, a (possibly approximate) inverse mapping of the transformation may be used to (at least approximately) project the prediction coefficients onto the data set dimensions. In case of a linear transformation, the inverse mapping may be the inverse found by matrix inversion. In some cases, such as PCA, since the eigen vectors in the matrix of eigen vectors are orthogonal, the inverse mapping may be the transpose of the linear transformation or the linear transformation itself. In some implementations the transformation may be non-linear and the inverse operation of the transformation, the inverse mapping that maps from embedded data samples to corresponding data samples, may be used to at least approximately project or convert the prediction coefficients to the data set dimensions. The inverse mapping may be approximate, for example found by numerical optimisation.

The embedded data set is then applied as an input to the predictor to produce a predicted value of the time-varying property for each embedded sample and prediction coefficients of the predictor are adjusted to reduce a measure of prediction error between respective predicted and actual values of the time-varying property. In some implementations, the predictor is also obtained without use of any gene pathway or biological activity information. In some implementations, a predictor may be obtained in this way in the first place and may then be refined using prior knowledge of gene pathways or biological activity, or biological knowledge derived from the prediction coefficients of the predictor.

In some implementations, the embedded data set may be scaled to have substantially constant variance across dimensions. This boosts the initial contribution of lower variance dimensions of the embedded data set to the adjusting of the prediction coefficients, as opposed to unweighted PCA regression, for example. The inventor has realised that high variance components do not necessarily relate to the time-varying property but instead may represent other sources of biological or technical variation. By weighting the variability of all components equally, lower variance components have an equal starting point in the regression optimisation, which may facilitate uncovering biologically relevant components.

In some implementations, the predictor is a linear predictor. Advantageously, this enables the prediction coefficients to be readily interpretable, for example as described above. The linear predictor may in some implementations comprise a regularisation method to promote sparseness of the prediction coefficients, which can further help with the interpretability, as fewer coefficients will make a significant contribution to the prediction. For example, in some implementations, adjusting the prediction coefficients comprises elastic net regression. In some implementations, the prediction error may be a median absolute prediction error.

Some implementations involve receiving a further data set. The further data set comprises further data samples obtained from respective further cell samples having different values of the time-varying property, each further data sample comprising a number of further transcription levels, and a respective further actual value of the time-varying property of the further cell sample for each further data sample. The further transcription levels have been derived from further transcription counts of gene transcripts in the further cell samples without use of knowledge of gene pathways, as discussed above. These implementations further involve transforming the data set and the further data set into a common data set comprising the data samples and the further data samples wherein transforming the data set and the further data set comprises reducing variability of the data samples and further data samples that is not common to the data set and the further data set. In these implementations, generating the embedded data set comprises generating for each data sample in the common data set an embedded sample.

Some implementations specifically enable predicting time-varying properties for new data sets by using a labelled data set to predict properties for an unlabelled one. These implementations also involve receiving a further data set but in this case without the time-varying property values. Again, transforming the data set and the further data set into a common data set comprising the data samples and the further data samples comprises reducing variability of the data samples and further data samples that is not common to the data set and the further data set and generating the embedded data set comprises generating for each data sample in the common data set an embedded sample. In these implementations, applying the embedded data set as an input to the predictor comprises applying only the embedded samples corresponding to the gene transcription data samples as an input to produce respective predicted values of the time-varying property for the embedded data samples corresponding to the data samples. After obtaining the predictor, these implementations include applying the embedded samples corresponding to the further data samples to the predictor to predict respective values of the time-varying property for the further cell samples.

Described implementations may further comprise generating a report that identifies a value of the time-varying property of one or more individuals or subjects for which cells have been obtained and/or an indication of the contribution to the prediction of the genes or transcripts in the data set. The report may be stored in any suitable form, for example digitally on a storage medium or media, may be displayed on a display screen and/or printed on paper or another suitable medium.

The disclosure further extends to a computer program product comprising computer code instructions that, when executed on a processor, implement the described methods and in particular those described above, for example computer readable medium or media comprising such computer code instruction. The disclosure further extends to systems comprising a processor and such a computer readable medium, wherein the processor is configured to execute the computer code instructions and to systems comprising means for implementing described methods, in particular those as described above.

BRIEF DESCRIPTION OF THE DRAWINGS

The following description of specific implementation is made by way of example to illustrate at least one disclosed invention and with reference to the accompanying drawings. Headings are included for clarity of exposition only and are not to be used in any interpretation of the content of the disclosure. In the drawings:

Figure 1 illustrates a computer-implemented method of obtaining a predictor for a time-varying property for cell samples;

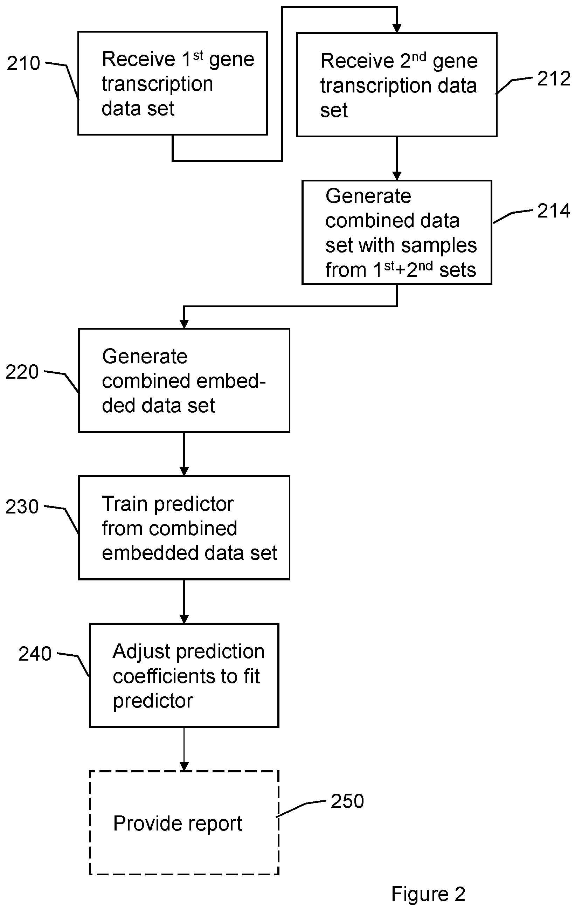

Figure 2 illustrates a computer-implemented method of obtaining a predictor for a time-varying property for cell samples comprising merging data sets corresponding to different batches of cell samples;

Figure 3 illustrates a computer-implemented method of obtaining a predictor for a value of a time-varying property for cell samples comprising merging data sets corresponding to different batches of cell samples and using the predictor obtained from one batch to predict the time-varying property for another batch;

Figure 4 illustrates an example hardware implementation suitable for implementing disclosed methods;

Figure 5 shows the distributions of median absolute error (MAE) in predicted age of aging clocks, trained using varying numbers of principal components as the input;

Figure 6 **A** to **G** demonstrate the performance of clocks trained directly on gene expression ("Expr. Clock") and by the method described herein ("RD clock") in single cells of the test set from various mouse organs;

Figure 7 shows the average time taken to perform a single iteration of clock training vs. the number of cells used for the training process; and

Figure 8 illustrates a comparison of performance metrics for different aging clocks.

DETAILED DESCRIPTION

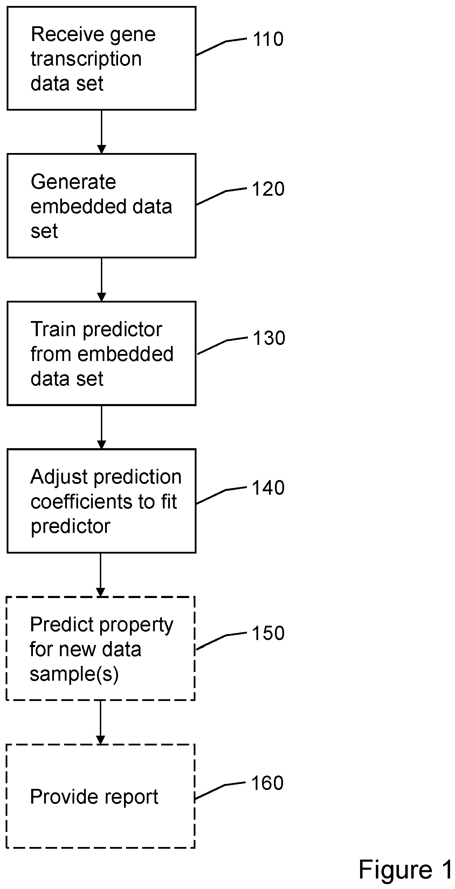

With reference to Figure 1, a gene transcription data set is received at step 110 for a batch of cell samples. The cell samples and data set may have been obtained in any suitable way, for example as described above. The data set is generated from raw gene transcription counts (counts of individual transcripts or summed by gene), one count per transcript or gene, cell sample and measurement time point to give one expression vector of expression levels per cell sample and time point. A data sample may be obtained from counts of a single cell (single cell sample) or may be obtained from pooled counts of a sample of several cells. The data set is or has been processed to derive expression levels using conventional numeric conditioning of the count data, including normalising the data, log transforming the data, and normalising the log transformed data to have zero mean and unity standard deviation, for example, noting that overall scale factors can of course be varied at will. Crucially, each transcription level therefore is of an individual gene transcript or of gene transcripts of an individual gene pooled, for example summed, for that gene. As a result, no prior knowledge of gene pathways or biological activity is needed in the processing to generate the expression vectors. Further, the subsequent described processing may be done without using prior knowledge of gene pathways or biological activity. It will, of course be understood that additional processing steps, such as postregression further adjustment of prediction coefficients may use such prior knowledge or may use such biological knowledge as is obtained from the regression itself, for example about how predictive certain genes are of the time-varying property, which can for example be derived from prediction coefficients, as described below.

The term "gene pathways" refers to networks of genes that function together to perform a particular biological process. Such a biological process can also be referred to as a "biological pathway", i.e. a series of interactions amongst molecules in a cell that result in a biological effect, for example a change in the cell or the production of a product. Those molecules are encoded by genes and it can therefore be seen that the result of the network of genes in a gene pathway will be a biological pathway. Knowledge on gene pathways and biological pathways can be obtained e.g. from the "Hallmark" pathway collection (Liberzon, A. et al. The molecular signatures database hallmark gene set collection. Cell Syst. 1, 417-425 (2015)) or publicly available databases such as the KEGG pathway database (https://www.kegg.jp/).

The resulting gene transcription data set is organised (or received) as a matrix E having the transcription vectors as row vectors with one row per cell sample and time point. An eigenvector matrix $\mathbf{W}$ and diagonal matrix of eigenvectors (variances of $\mathbf{E}$ transformed with basis vectors $\mathbf{W}$) is found using any suitable technique such as eigen decomposition or more typically singular value decomposition.

$$\mathbf{E}^{\mathrm{T}}\mathbf{E}\mathbf{W} = \mathbf{W}\Lambda \qquad \text{Eq. 1}$$

An embedded data set $\mathbf{X}$ is formed at step 120 using the matrix $\upsilon$ of k column eigenvectors (or principal components) $\mathbf{w_i}$

$$\upsilon = \begin{bmatrix} \mathbf{w_1} & \mathbf{w_2} & \mathbf{w_3} & \cdots & \mathbf{w_k} \end{bmatrix} \qquad \text{Eq. 2}$$

associated with the largest eigenvalues (or variance explained) $\Lambda_{i,i}$ and a diagonal scaling matrix $\mathbf{S}$ that scales the principal components by their inverse standard deviation across cell samples in order to level the playing field in the initial contribution to the regression between the higher and lower variance components of the principal components, as discussed above.

$$\mathbf{X} = \mathbf{E}\upsilon\mathbf{S}; \ \ \mathrm{diag}(\mathbf{S})_i = \frac{1}{\sqrt{\Lambda_{i,i}}} \qquad \text{Eq. 3}$$

$k$ can be chosen as suitable, with higher values requiring more computation but potentially including more biologically relevant information. $k = 50$ has been found to be a suitable maximum value in most settings, and in some implementations $k$ may be, for example, between 20 and 30. $k$ can also be chosen in an iterative way by comparing performance of the coefficient fitting described below for different values of $k$ and choosing a value that achieves the best or at least satisfactory performance. In some implementations, instead of selecting the components with the $k$ largest eigenvalues, components can be selected according to different criteria, for example in the middle of the range of eigen values or at specific ordinals of eigen values, in some implementations based on performance as described above.

Each data sample of the data set further comprises an actual value of a time-varying property of the cell-sample (or the organism from which the cell sample is obtained), noting that the data set contains multiple cell samples at multiple time points and that there is one such value per cell sample and time point. The actual values may be measured at each time point, for example by measuring a quantity such as a biomarker indicating biological age or correlated with a disease trajectory, may be separately known for the organism, such as a disease progression or stage, or may simply be the time point itself, as in the case of chronological age. Aging clock measurements such as epigenetic clock measurements can be used as a biomarker indicating biological age. In addition to biological or chronological age or the stage or progression of a disease of condition, for example a neurodegenerative condition such as Alzheimer's or Parkinson's disease, any other time-varying property of the cell sample or organism from which it was derived may be used.

The actual values are organised or received in a column vector $y$ having the same number of rows as $\mathbf{E}$, one for each data sample. A linear predictor

$$\mathbf{y}^\star = \mathbf{X}\boldsymbol{\beta} + \beta_0 \qquad \text{Eq. 4}$$

is trained for the embedded data set X by applying at step 130 the embedded data set to the predictor to predict a value **y\*** of the time-varying property $y$ by adjusting the prediction coefficients in vector $\beta$ containing the linear weights for the principal components in the regression and the offset $\beta_0$ at step 140. The coefficients are adjusted to minimise a measure of the error between y and **y\***, for example the mean of the squared error $\overline{(y^* - y)^2}$ or the median of the absolute error $\widetilde{|\mathbf{y^* - y}|}$. Various minimisation methods may be used, including simple least square regression. In some implementations, it has been found advantageous to use elastic net linear regression (see Zou, H., & Hastie, T. (2005) Regularization and variable selection via the elastic net; Journal of the Royal Statistical Society: Series B (Statistical Methodology), 67(2), 301-320; https://doi.org/10.1111/j.1467-9868.2005.00503.x, incorporated by reference herein, which also discusses several alternative regression approaches that may be used in some implementations). Advantageously, elastic net regression promotes sparsity of the prediction coefficients, that is the coefficients tend to be small for most coefficients with the mass of coefficients being concentrated in more predictive regression variables (in this case more predictive principal components). This facilitates the interpretability of the principal components (and hence the corresponding transcription levels) in terms of their biological relevance in the process captured by the time-varying property.

The training and adjusting of coefficients may be implemented in any suitable manner. To reduce overfitting issues, it can be advantageous to train the parameter using n-fold crossvalidation. Additionally, some data may be held back as pure test data to assess model performance on unseen data. Any linear predictor may be used in dependence on specific implementations and may combine the embedding and regression steps. One linear predictor that may be used is partial least squares or variants thereof, which include an embedding of both **E and y.** The present disclosure is, however, not limited to linear predictors and other predictors, such as feedforward or recurrent neural networks may be used to provide a predictor of values of the time-varying property. It is noted that linear predictors are advantageous not only for their algorithmic simplicity and efficiency but also due to the interpretability of the prediction coefficients, as discussed below.

To assess the contribution of each expression level to the prediction of the time-varying property, that is determine which expression levels are more predictive than others, the prediction coefficients may be projected back to contribution coefficient $\beta^*$ in the space of the expression levels by

$$\boldsymbol{\beta^* = \upsilon R \beta}; \ \ \mathrm{diag}(\mathbf{R})_i = \sqrt{\Lambda_{i,i}} \qquad\qquad \text{Eq. 5}$$

where **R** "unscales" the coefficients to compensate for the scaling by **S** during regression. The elements of $\beta^*$ thus provide a measure of how predictive the gene or transcription corresponding to the respective transcription level is for the time-varying property.

At an optional step 150, a new transcription sample may be received and the trained predictor may be used to predict a value of the time-varying property of the new transcription sample. The new transcription sample may be a sample obtained from the same experiment/event or set of experiments/events used to obtain the samples used for training, for which a value of the time-varying property is not available, or the new transcription sample may be newly obtained. For a good prediction, the conditions under which the newly obtained sample is obtained must be carefully controlled to match those under which the training samples were obtained to avoid significant batch effects due to difference in technical noise degrading prediction performance. In many cases, this can be a challenge and the following discusses methods correcting for such batch effects, either to add new training data to existing training data or to combine unlabelled new data with the training data set or sets to improve prediction performance.

At a further optional step 160, a report may be generated providing one or both the elements of $\beta^*$ for each gene / transcript to allow their predictiveness to be assessed and a predicted value of the time-varying property for one or more new data samples, if applicable. Other elements of the report may be regression coefficient or other indicators of goodness of fit, residuals and/or any other quantity that may facilitate the interpretation of the data and of the predictor.

A process for training a predictor and making predictions using a combined data set comprises a step 210 of receiving a first gene transcription data set **E** and a step 212 of receiving a second (further) gene transcription data set **Ẽ**, each as described above for step 110. The two data sets are combined into a combined data set

$$\mathbf{C} \leftarrow \{\mathbf{E}|\tilde{\mathbf{E}}\} \qquad\qquad \text{Eq. 6}$$

at step 214 where $\{\cdot|\cdot\}$ is a data set combination operation, in the simplest implementation a concatenation of the two data sets. In some implementations, the combination operation comprises a suitable normalisation of the individual data

$$\left( e_n \leftarrow \frac{e_n}{\|e_n\|} \right).$$

sets, for example replacing the expression levels with their cosine norm computed for each cell sample

In some implementations, the data set combination operation includes a correction for differences (typically due to technical noise) between data sets of different batches. In some implementation, a batch correction vector is subtracted from each data sample in the second batch, or in terms of a batch correction matrix $\mathbf{B}$ of batch correction row vectors

$$\mathbf{C} \leftarrow \begin{bmatrix} \mathbf{E} \\ \tilde{\mathbf{E}} - \mathbf{B} \end{bmatrix} \qquad \text{Eq. 7}$$

The embedded data set X can then be formed at step 220 in analogous fashion to step 120 as

$$\mathbf{X} = \mathbf{C}\bar{\upsilon}\bar{\mathbf{S}}; \quad \mathrm{diag}(\bar{\mathbf{S}})_i = \frac{1}{\sqrt{\bar{\Lambda}_{i,i}}} \qquad \text{Eq. 8}$$

where $\bar{\upsilon}$ and $\bar{\Lambda}_{i,i}$ are, respectively, the eigen vectors / principal components and the eigen values / variances explained of $\mathbf{C}$ in place of $\mathbf{E}$ in step 120 and equation 3. Steps 230 of training the predictor, 240 of adjusting the prediction coefficients and 250 of providing a report are then analogous to steps 130, 140 and 160 described above, and the corresponding disclosure applies accordingly. Advantageously, by combining data sets from different batches, for example from different experiments, different instances of the same experiment over time, different individuals of a particular organism and so forth, richer data sets can be created and used to obtain improved predictors.

In some implementations, combining the data sets at step 214, equations 6 and 7, comprises transforming the data sets to a different coordinate system. In specific implementations, the principal components of the combined data set are found, and the combined data set is transformed using the matrix $\hat{\upsilon}$ of the principal component of the combined data set associated with the k largest eigenvalues

$$\begin{bmatrix} \mathbf{E} \\ \tilde{\mathbf{E}} \end{bmatrix} \leftarrow \begin{bmatrix} \mathbf{E} \\ \tilde{\mathbf{E}} \end{bmatrix} \hat{\upsilon} \qquad \text{Eq. 9}$$

and the transformed data sets are then used as described above

Computing the principal components of the combined data set comprises centring on the average of the means of each data set to be merged (rather than just on the mean of the combined data set) and weighting the contribution of each cell sample to the covariance matrix by the inverse of the number of cell samples in the respective data set to be merged (or, equivalently, by using the average of the covariance matrices of the data sets to be merged as the covariance matrix for the principal component analysis). Principal components are then computed for the combined data set in the conventional manner, for example using eigen or singular value decomposition.

Batch correction then proceeds as described above with reference to equations 6 and 7 on the selected principal components of the combined data set. In these implementations, the dimensions of the combined data set remain orthogonal through the batch correction and while equation 8 can be used to form the combined embedded data set X, there is no need to do so and the same selected k dimensions of $\mathbf{C}$ can be used to form the combined embedded data set

$$\mathbf{X} = \mathbf{C}\bar{\mathbf{S}}; \quad \mathrm{diag}(\bar{\mathbf{S}})_i = \frac{1}{\sqrt{V_{i,i}}} \qquad \text{Eq. 10}$$

where $V_{i,i}$ are the non-zero diagonal entries of the covariance matrix $\mathbf{V}$ of $\mathbf{C}$. Naturally, a smaller number of dimensions of C can be selected.

Various approaches for calculating the batch correction vectors $\mathbf{B}$ are known and may be used in implementations. In some implementations, a mutual nearest neighbour (MNN) approach is used, see Haghverdi, L., Lun, A. T. L., Morgan, M. D., & Marioni, J. C. (2018) Batch effects in single-cell RNA-sequencing data are corrected by matching mutual nearest neighbors; Nature Biotechnology, 36(5), 421-427; https://doi.org/10.1038/nbt.4091 and https://marionilab.github.io/FurtherMNN2018/theory/description.html, each of which is incorporated by reference herein. MNN are defined by first creating a list of $K$ nearest neighbours for each $\mathbf{E}_n$ in $\tilde{\mathbf{E}}$ and second list of K nearest neighbours for each

$\tilde{\mathbf{E}}_{\tilde{n}}$ in **E.** Two cell samples n and n in the respective data sets are MNN if n is found in the list for $\tilde{n}$ and n is found in the list for $\tilde{n}$. K is chosen based on experience or empirically for each dataset with a larger number of nearest neighbours increasing robustness to noise and sampling nearest neighbours deeper into each cloud of cell samples but increasing computational cost. In practice K = 20 is a suitable choice.

MNN batch correction vectors for MNN are the difference vector $\mathbf{E}_n$ - $\tilde{\mathbf{E}}_{\tilde{n}}$. In implementations in which MNN are found directly based on expression levels without an orthogonalisation and/or dimensionality reduction, such as PCA, as described above. In these implementations, MNN may be found using highly variable genes (HVG), as is common in the field. While MNN may be found using HVG in some implementations, all genes of interest or all genes available may be included at this stage of computing batch correction vectors or separate batch correction vectors may be computed for each set of genes of interest.

The above results in a set of batch correction vectors for the MNN, or MNN batch correction vectors. Batch correction vectors for other data samples that are not MNN are then found from the MNN batch correction vectors, for example by combining them with a Gaussian kernel, using another form of weighted average, using only MNN batch correction vectors of nearest neighbours of each cell sample, and so forth. This provides locally varying batch correction vectors for all data samples, that are then used in equation 7 as described above.

In some implementations, the cell samples in each batch are projected onto a respective bisecting plane perpendicular to an average vector of the MNN batch vectors in each data set prior to applying the MNN batch vectors as described above (but adjusted for the projection of the MNN cell samples themselves). This ensures that the merged cell samples intermingle and are not just brought together as touching clouds, even if K is not large enough to sample nearest neighbours beyond the notional facing surfaces of the batches. Alternatively, the cell samples in the merged data set after batch correction can be projected into a common bisecting plane perpendicular to the average MNN batch correction vector or this step can be omitted, in particular for sufficiently large values of K.

Full details of a method of batch correction in line with the above are set out in Haghverdi *et al.* (2018) cited above, with supplementary information and software packages available as part of the *batchelor* R package, also described in Haghverdi *et al.* (2018). See https://marionilab.github.io/FurtherMNN2018/theory/description.html for a further, related implementation that squashes variation along the average MNN batch correction vector in each data set prior to applying the batch correction vectors, as described above. Alternative methods of batch correction that output a reduced dimensionality embedding of the corrected dataset may equally be used, for example Seurat v3, which implements canonical correlation analysis before identification of "anchors" in a similar manner as above.

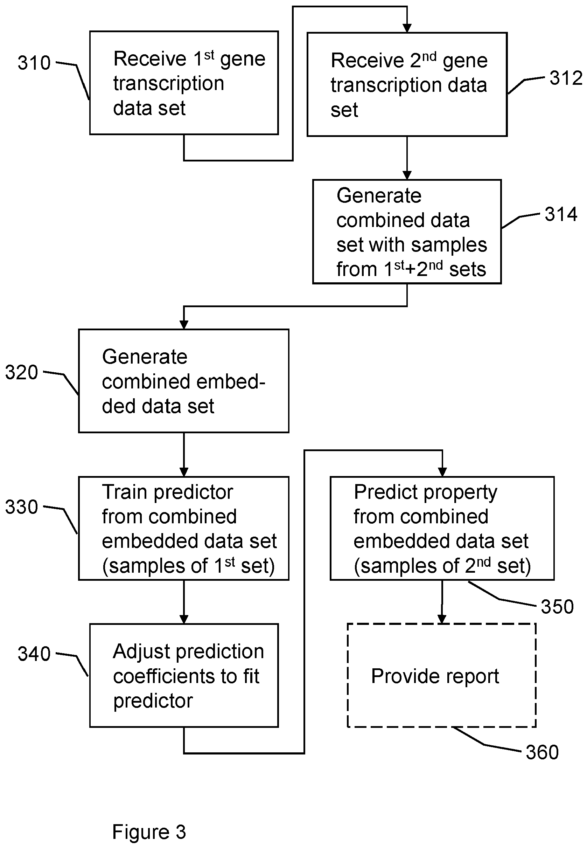

With reference to Fig 3, the steps of receiving the first and second gene transcription data sets 310, 312, generating the combined data set 314, generating the combined embedded data set 320, training the predictor 330 and adjusting the prediction coefficient 340 are analogous to steps 210, 212, 214, 220, 230 and 240 described above and the corresponding disclosure applies accordingly, with the exception that only the first gene transcription data set comprises actual values y of the time-varying property and this information is not received (or is ignored) with the second gene transcription data at step 312. Accordingly, the predictor is trained and the prediction coefficients adjusted at steps 330 and 340 using only the data samples from the first data set for which the property values are available and the resulting predictor is then used to predict respective values of the property for data samples of the second data set. In this way, unknown values of the property can be predicted, for example for samples obtained from a new individual of an organism, for which the time-varying property is not known. A step 360 of preparing a report is analogous to step 160 described above, including the predicted value(s) for the sample(s) in the second data set.

The described implementations compute an embedding using principal component analysis, for example implemented using SVD, and select a number of principal components for dimensionality reduction. Other methods of obtaining an embedding are equally applicable in various implementation and can be used to replace PCA for the embedding. For example, the embedding may be found using non-linear methods such as kernel methods, for example kernel PCA (kPCA) or non-linear methods such as training an autoencoder (AE). kPCA applies eigen decomposition or SVD to a kernel matrix derived from the data using a kernel function in a similar way as PCA does to the covariance matrix. The prediction coefficients of genes can be recovered in a similar way to the one described for PCA above, finding the weightings in gene space using an inverse mapping. The inverse mapping may be found by numerical optimisation and the resulting gene prediction coefficients may be recovered at least approximately. AE are neural networks that are trained to match their input at their output and comprise a hidden embedding layer with less units than then in and output layers that provides the embedding. Gene prediction coefficients can be at least approximately recovered from the embedded prediction coefficients using the trained decoding network between the hidden embedding layer and the output layer of the network. In general, at least approximate gene prediction coefficients can be found from the embedded prediction coefficient by applying an inverse mapping of the embedding transformation to the embedded prediction coefficients. The inverse mapping may correspond to a mathematical inverse or may be any other operation mapping from the embedded to the gene space, that is from embedded data samples to the corresponding data samples. This projection onto the dimensions of the (non-embedded) data set may thus be approximate (for example found by numerical methods or neural network training) or mathematically exact (for example found by matrix inversion or transposition as in the case of PCA as the embedding, described in detail above).

EXAMPLE HARDWARE IMPLEMENTATIONS

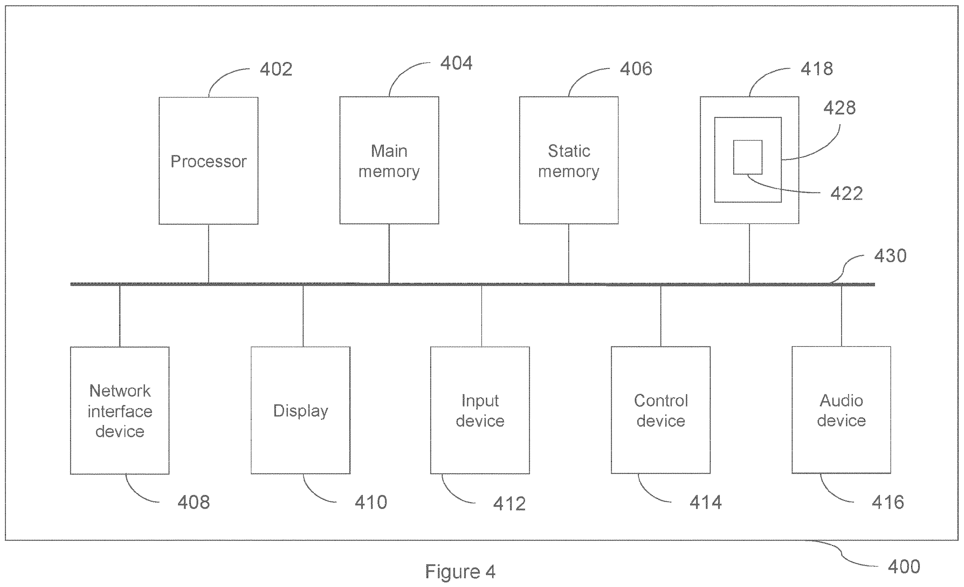

Figure 4 illustrates a block diagram of one implementation of a computing device 400 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 400 includes a processing device 402, a main memory 404 (e.g., read-only memory (ROM), flash memory, dynamic random access memory (DRAM) such as synchronous DRAM (SDRAM) or Rambus DRAM (RDRAM), etc.), a static memory 406 (e.g., flash memory, static random access memory (SRAM), etc.), and a secondary memory (e.g., a data storage device 418), which communicate with each other via a bus 430.

Processing device 402 represents one or more general-purpose processors such as a microprocessor, central processing unit, or the like. More particularly, the processing device 402 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 402 may also be one or more special-purpose processing devices such as an application specific integrated circuit (ASIC), a field programmable gate array (FPGA), a digital signal processor (DSP), network processor, or the like. Processing device 402 is configured to execute the processing logic (instructions 422) for performing the operations and steps discussed herein.

The computing device 400 may further include a network interface device 408. The computing device 400 also may include a video display unit 410 (e.g., a liquid crystal display (LCD) or a cathode ray tube (CRT)), an alphanumeric input device 412 (e.g., a keyboard or touchscreen), a cursor control device 414 (e.g., a mouse or touchscreen), and an audio device 416 (e.g., a speaker).

The data storage device 418 may include one or more machine-readable storage media (or more specifically one or more non-transitory computer-readable storage media) 428 on which is stored one or more sets of instructions 422 embodying any one or more of the methodologies or functions described herein. The instructions 422 may also reside, completely or at least partially, within the main memory 404 and/or within the processing device 402 during execution thereof by the computer system 400, the main memory 404 and the processing device 402 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid-state memory, magnetic tape, a removable computer diskette, a random-access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing", "enabling", "maintaining," "identifying, "obtaining", "receiving", "generating", "applying", "adjusting", "producing", "scaling", "deriving" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

EXAMPLES

To demonstrate the aging clock method, the inventors analysed single-cell gene expression data from the Tabula Muris Senis (The Tabula Muris Consortium. A single-cell transcriptomic atlas characterizes ageing tissues in the mouse. Nature 583, 590-595 (2020). https://doi.org/10.1038/s41586-020-2496-1, incorporated by reference), containing the transcriptomes of cells of multiple tissues from mice with known chronological age. The data obtained from the microfluidic ("droplet") method contained four tissues with sufficient timepoints to attempt clock training: the heart, lung, limb muscle and spleen. Within these tissues, the most prevalent cell types (annotated by the original authors) were selected, leading to the selection outlined in Table 1. Analysis was also limited to male mice to prevent any sex effect.

Table 1 - A table outlining the tissues and their contributing cell types in the Tabula Muris Senis for which there are sufficient cells to reliably train a single-cell aging clock.

| Tissue | Suitable cell types |
|---|---|
| Heart | Fibroblast, Endothelial cell |
| Lung | Classical monocyte, Bronchial smooth muscle cell |
| Limb muscle | Mesenchymal stem cell, Skeletal muscle satellite cell |
| Spleen | B cell |

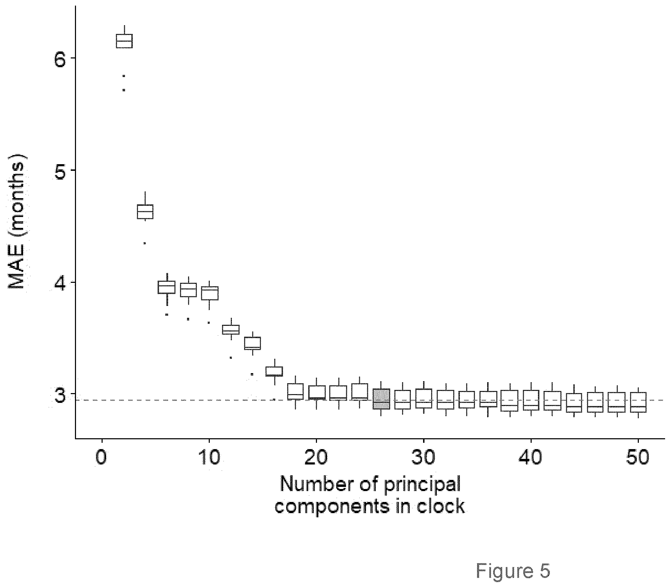

The median absolute error (MAE) was found to be a good loss function for the training process described herein. Figure 5 is an example showing the distributions of MAE in predicted age of aging clocks, trained using varying numbers of principal components from heart endothelial cells as the input. In clocks trained on fewer principal components, the latter-most have been discarded. The training process was repeated 10 times for each number of principal components. The dotted line is the threshold for a near-optimal model, and the shaded box is the lowest number of principal components that can produce a clock with this performance. The threshold was calculated by first identifying the number of principal components that produce a clock with the lowest mean MAE. The standard error of the MAEs produced by the 10 clocks trained using this number of principal components was then added to the mean MAE of this group of clocks.

Clock testing

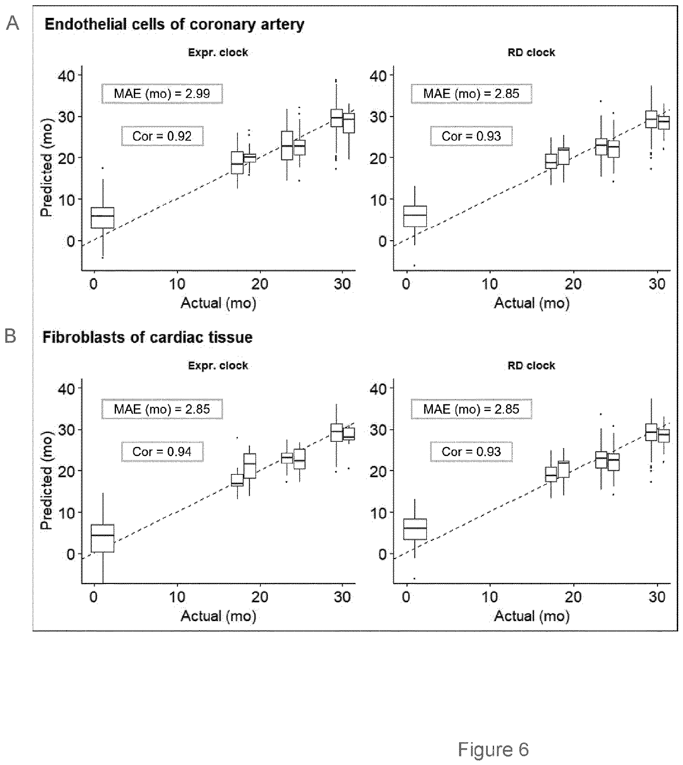

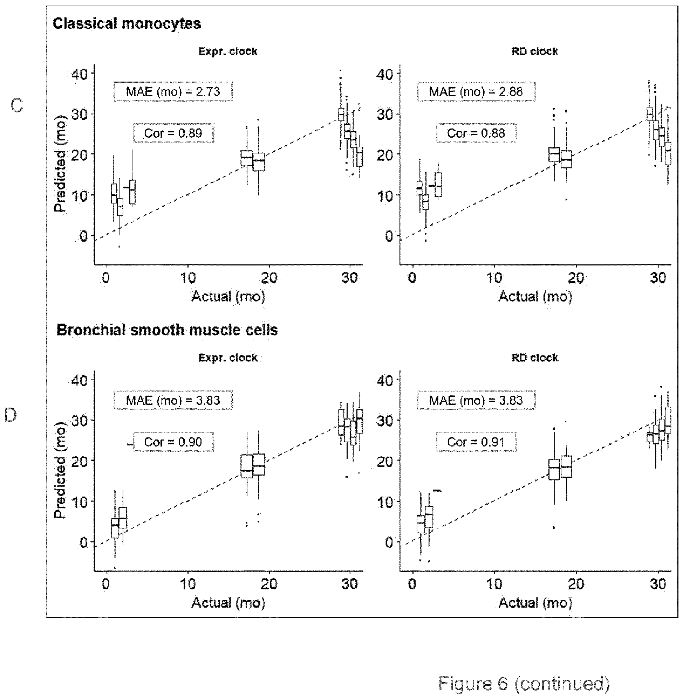

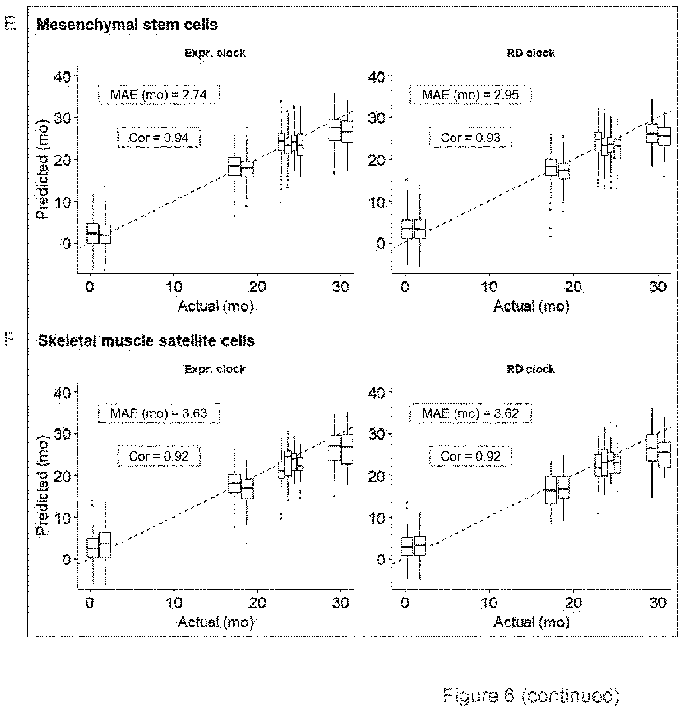

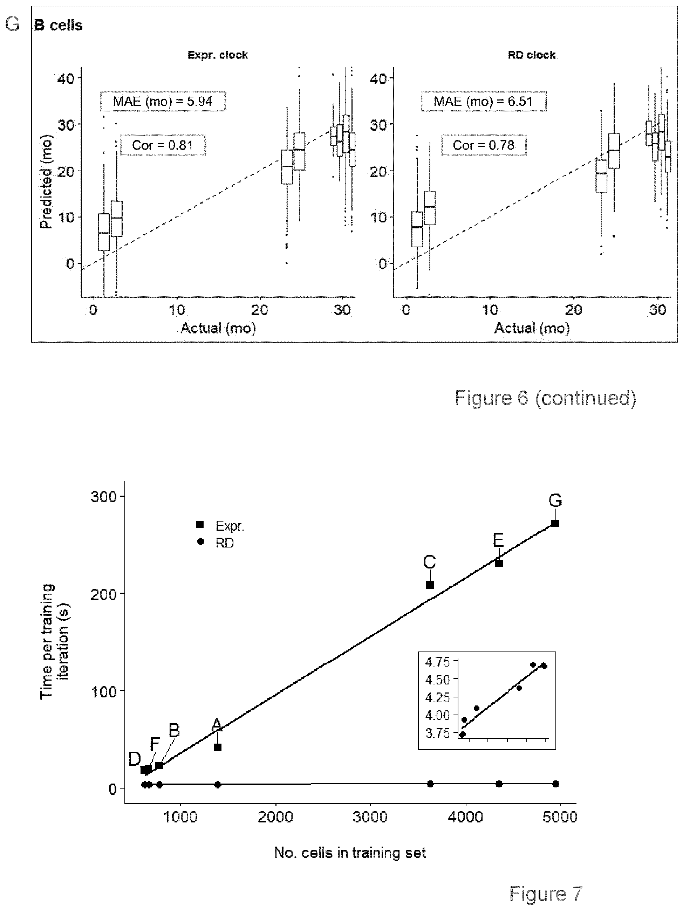

Figure 6 **A** to **G** illustrate the performance of clocks trained directly on gene expression and by the method described herein (referred to in the Figures as "RD clock") in single cells of the test set from mouse heart (**A** and **B),** lung (**C** and **D),** limb muscle (**E** and **F**) and spleen (**G);** the individual cell types are labelled as follows.

| Letter | Tissue: cell type |
|---|---|
| A | Heart: endothelial |
| B | Heart: fibroblasts |
| C | Lung: classical monocytes |
| D | Lung: bronchial smooth muscle |
| E | Limb muscle: mesenchymal stem cells |

(continued)

| Letter | Tissue: cell type |
|---|---|
| F | Limb muscle: skeletal muscle satellite cells |
| G | Spleen: B cells |

Each boxplot represents the distribution of predicted ages for cells from a single donor mouse: grouped boxplots correspond to mice of the same age, jittered in the x direction to aid visualisation. The upper and lower hinges correspond to the 75th and 25th percentiles, respectively, with the middle hinge denoting the median value. Whiskers extend 1.5 * interquartile range from the outer hinges, and points falling outside this range are represented by black points. The median absolute error (MAE) per cell is shown in months for each plot, as is the Pearson correlation coefficient (Cor.) and y = x is denoted by a dashed black line.

When trained and tested within a single dataset (i.e. where there is not batch effect present between the training and test cells), the error of the method described herein (measured by MAE) is similar to that of a clock trained directly on the top 2000 highly variable genes, as can be seen from Fig. 6A to G. However, as later principal components have been discarded from the clocks described herein, the models will be contributed to less by technical noise and are likely to be less biased by "overfitting". Thus, the accuracy of the clocks described herein is less inflated than that of direct gene expression clocks.

As can be seen from Fig. 7, another benefit is the reduced time required for clock training and this time reduction scales with increasing number of cells used for training. Figure 7 shows the average time taken to perform a single iteration of clock training (directly on gene expression ["Expr.", squares] or by the method described herein ["RD", circles]) is shown vs. the number of cells used for the training process. The points are lettered according to the tissue and cell type used for training, and a straight line has been fitted using linear regression. Inset: the values from the clock method are shown with an identical x-axis to the main plot, but with a truncated y-axis to aid visualisation. Training was performed using an AMD Ryzen 7 5800X 8-Core Processor (3.80 GHz) and 32 GB RAM.

This time reduction is significant, given that the training process often needs to be repeated thousands of times during training and optimisation. For reference, with a "realistic" training set size of -5000 cells (spleen B cells), the method described herein is approx. 60 times faster.

Transferring clock between datasets

The aging clock method can be used to predict the age of single cells' donors in a dataset for which there is little or no prior age annotation. The inventors once more used the Tabula Muris Senis to demonstrate this, as it also contains single-cell expression data for the four previously used tissues, collected by a different sequencing method based on fluorescence activated cell sorting (FACS). The cells of male mice were collected at 3, 18 and 24 months. However, given the lack of male mouse samples between 1 and 18 months in all tissues profiled by the droplet method, 3-month-old cells were excluded from further analysis.

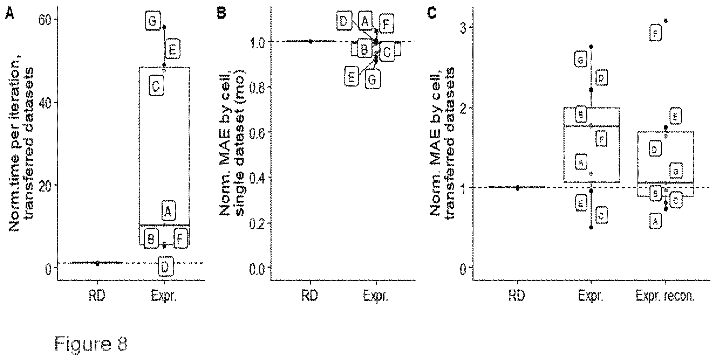

Figure 8 shows a comparison of performance metrics for an aging clock as described herein ("RD") and a clock trained directly on gene expression ("Expr."). In each panel, the metrics are normalised to that of the clock described herein. **A:** the time taken per ELN training iteration in a single dataset; **B:** MAE per cell when the clocks are trained and tested on a single dataset; **C:** MAE per cell when the clock is trained in one dataset and used to predict the age of cells in a separate dataset. In **C,** the clock described herein was trained on the corrected PCA matrix produced by the MNN method for droplet cells and tested on FACS cells; the direct expression clocks trained in Figure 6 **A** to **G** were applied directly to FACS cells; a clock was also trained on the expression matrix reconstructed from the MNN-corrected PCA matrix ("Expr. recon.").

As set out above, the direct expression clocks previously trained on droplet data were applied to the FACS data; on average they performed worse than clocks described herein trained on the droplet data and tested on FACS data, after batch correction (Fig. 8C). The mean reduction of MAE in the clocks described herein was 37%; this improvement is also likely to be much larger in datasets where the batch effect is more significant.

Overfitting to technical noise will also contribute to the increased error of direct expression clocks relative to clocks described herein when they are batch-transferred. In general, overfitting will reduce the generalisability of direct expression clocks, and any biological conclusions derived thereof. This means that a direct expression clock trained in a dataset would perform poorly in a biological replicate of that datasets, even in the (highly unlikely) case of absolutely zero batch effect. As the latter condition is hard to satisfy, one way in which the generalisability of these clocks can be investigated is to use the output of batch correction. As the output of the MNN method is a corrected matrix analogous to a corrected PCA matrix, a "corrected" gene expression matrix can be reconstructed from this matrix by a similar method to that described herein. It is important to note that, due to the forced movement of cells in PCA space, the resulting "expression" matrix will be highly distorted and should not generally be used as a mathematical substitute for real gene expression. However, this matrix represents the only practical method by which the generalisability of a clock as described herein can be compared to a direct expression clock in the absence of a batch effect. Under these conditions, the clock method described herein yielded reduced error (mean MAE reduction = 30%, Fig. 8C), suggesting that significant benefit arises from the lack of overfitting in clocks according to the disclosure.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

Disclosed aspects and embodiments include the following numbered clauses:

1. A computer-implemented method of obtaining a predictor for predicting a time-varying property based on gene transcription data, the method comprising:

receiving a data set comprising data samples obtained from respective cell samples having different values of the time-varying property, each data sample comprising a number of transcription levels, and a respective actual value of the time-varying property of the cell sample for each data sample, wherein each transcription level is a transcription level of an individual gene transcript or a pooled transcription level of gene transcripts of an individual gene;

generating an embedded data set comprising for each data sample an embedded sample, wherein a number of dimensions of the embedded samples is less than the number of transcription levels;

applying the embedded data set as an input to the predictor to produce a predicted value of the time-varying property for each embedded sample;

obtaining the predictor by adjusting prediction coefficients of the predictor to reduce an error measure of prediction error between respective predicted and actual values of the time-varying property.

2. The method of clause 1 comprising applying a transformation to the data set to generate the embedded data set, the method further comprising obtaining the transformation by operating on the data set.

3. The method of clause 2 comprising obtaining the transformation without using knowledge of gene pathways.

4. The method of clause 2 or 3 comprising obtaining the transformation by operating on a covariance matrix of the data set.

5. The method of any preceding clause, comprising scaling the embedded data set to have substantially constant variance across dimensions.

6. The method of any preceding clause, comprising applying a linear transformation to the transcription data set to generate the embedded data set.

7. The method of clause 6, wherein the embedded data set comprises a subset of the principal components of the transcription data set.

8. The method of any preceding clause, comprising applying an inverse mapping, mapping from the embedded data samples to the data samples, to the prediction coefficients to project the prediction coefficients onto the dimensions of the data set, thereby deriving a measure of contribution to predicting a value of the time-varying property for each gene or transcript.

9. The method of clause 8, when dependent on claims 6 and 7, wherein the inverse mapping comprises a matrix inversion of the linear transformation.

10. The method of any preceding clause, wherein the predictor is a linear predictor.

11. The method of clause 10, wherein the linear predictor comprises a regularisation method to promote sparseness of the prediction coefficients.

12. The method of any preceding clause, wherein adjusting the prediction coefficients comprises elastic net regression.

13. The method of any preceding clause, wherein the prediction error is a median absolute prediction error.

14. The method of any preceding clause, further comprising:

receiving a further data set comprising further data samples obtained from respective further cell samples having different values of the time-varying property, each further data sample comprising a number of further transcription levels, and a respective further actual value of the time-varying property of the further cell sample for each further data sample, wherein each further transcription level is a transcription level of an individual gene transcript or a pooled transcription level of gene transcripts of an individual gene;
transforming the data set and the further data set into a common data set comprising the data samples and the further data samples, thereby reducing variability of the data samples and further data samples that is not common to the data set and the further data set, and
wherein generating the embedded data set comprises generating for each data sample in the common data set an embedded sample.

15. The method of any one of clauses 1 to 14, further comprising:

receiving a further data set comprising further data samples obtained from respective further cell samples having different values of the time-varying property, each further data sample comprising a number of further transcription levels, wherein each further transcription level is a transcription level of an individual gene transcript or a pooled transcription level of gene transcripts of an individual gene;
transforming the data set and the further data set into a common data set comprising the data samples and the further data samples, thereby reducing variability of the data samples and further data samples that is not common to the data set and the further data set,
wherein generating the embedded data set comprises generating for each data sample in the common data set an embedded sample, and
wherein applying the embedded data set as an input to the predictor comprises applying only the embedded samples corresponding to the gene transcription data samples as an input to produce respective predicted values of the time-varying property for the embedded data samples corresponding to the data samples; and
after obtaining the predictor, applying the embedded samples corresponding to the further data samples to the predictor to predict respective values of the time-varying property for the further cell samples.

16. The method of any preceding clause, wherein the number of dimensions of the embedded samples is selected based on the respective prediction performance of embedded data sets having different respective numbers of dimensions.

17. The method of any preceding clause, wherein the time-varying property is of one or more organisms or subjects from which the cell samples have been obtained.

18. The method of clause 16, further comprising generating a report that identifies a value of the time-varying property for the one or more organisms or subjects.

19. The method of any one of clauses 1 to 16, wherein the cells have been obtained from cell culture.

20. The method of any preceding clause, wherein the cell samples are single cell samples of a single cell each.

21. The method of any preceding clause, wherein the time-varying property is biological age.

22. The method of any one of clauses 1 to 20, wherein the time-varying property is chronological age.

23. The method of any one of clauses 1 to 20, wherein the time-varying property is a state of progression of a condition or disease.

24. The method of clause 23, wherein the condition or disease is a neurodegenerative disease.

25. The method of clause 24, wherein the neurodegenerative disease is Alzheimer's disease.

26. The method of clause 24, wherein the neurodegenerative disease is Parkinson's disease

27. The method of any one of clauses 1 to 20, wherein the time-varying property is a state of progression of a cancer.

28. The method of any preceding clause, wherein the transcription levels and, where present, the further transcription levels have been derived from transcription counts of gene transcripts in the cell samples without use of knowledge of gene pathways.

29. The method of clause 28, comprising generating the embedded data set without use of knowledge of gene pathways.

30. The method of clause 28, comprising applying the embedded data set and obtaining the predictor without use of knowledge of gene pathways.

31. The method of any preceding clause, further comprising, after obtaining the predictor, refining the predictor using prior knowledge of gene path ways or biological activity, any other prior biological knowledge, or knowledge derived from the prediction coefficients.

32. Computer program product comprising computer code instructions that, when executed on a processor, implement the method of any preceding clause.

33. Computer readable medium or media comprising computer code instruction that, when executed on a processor, implement the method of any one of clauses 1 to 31.

34. System comprising a processor and a computer readable medium as defined in clause 33, wherein the processor is configured to execute the computer code instructions.

35. System comprising means for implementing a method as defined in any one of clauses 1 to 31.

**Claims**

1. A computer-implemented method of obtaining a predictor for predicting a time-varying property based on gene transcription data, the method comprising:

   receiving a data set comprising data samples obtained from respective cell samples having different values of the time-varying property, each data sample comprising a number of transcription levels, and a respective actual value of the time-varying property of the cell sample for each data sample, wherein each transcription level is a transcription level of an individual gene transcript or a pooled transcription level of gene transcripts of an individual gene;
   generating an embedded data set comprising for each data sample an embedded sample, wherein a number of dimensions of the embedded samples is less than the number of transcription levels;
   applying the embedded data set as an input to the predictor to produce a predicted value of the time-varying property for each embedded sample;
   obtaining the predictor by adjusting prediction coefficients of the predictor to reduce an error measure of prediction error between respective predicted and actual values of the time-varying property.

2. The method of claim 1 comprising applying a transformation to the data set to generate the embedded data set, the method further comprising obtaining the transformation by operating on the data set.

3. The method of claim 2 comprising obtaining the transformation without using knowledge of gene pathways.

4. The method of claim 2 or 3 comprising obtaining the transformation by operating on a covariance matrix of the data set.

5. The method of any preceding claim, comprising scaling the embedded data set to have substantially constant variance across dimensions.

6. The method of any preceding claim, comprising applying a linear transformation to the transcription data set to generate the embedded data set.

7. The method of claim 6, wherein the embedded data set comprises a subset of the principal components of the transcription data set.

8. The method of any preceding claim, comprising applying an inverse mapping, mapping from the embedded data samples to the data samples, to the prediction coefficients to project the prediction coefficients onto the dimensions of the data set, thereby deriving a measure of contribution to predicting a value of the time-varying property for each gene or transcript.

9. The method of any preceding claim, wherein the predictor is a linear predictor.

10. The method of any preceding claim, further comprising:

    receiving a further data set comprising further data samples obtained from respective further cell samples having different values of the time-varying property, each further data sample comprising a number of further transcription levels, and a respective further actual value of the time-varying property of the further cell sample for each further data sample, wherein each further transcription level is a transcription level of an individual gene transcript or a pooled transcription level of gene transcripts of an individual gene;
    transforming the data set and the further data set into a common data set comprising the data samples and the further data samples, thereby reducing variability of the data samples and further data samples that is not common to the data set and the further data set, and
    wherein generating the embedded data set comprises generating for each data sample in the common data set an embedded sample.

11. The method of any one of claims 1 to 10, further comprising:

    receiving a further data set comprising further data samples obtained from respective further cell samples having different values of the time-varying property, each further data sample comprising a number of further transcription levels, wherein each further transcription level is a transcription level of an individual gene transcript or a pooled transcription level of gene transcripts of an individual gene;
    transforming the data set and the further data set into a common data set comprising the data samples and the further data samples, thereby reducing variability of the data samples and further data samples that is not common to the data set and the further data set,
    wherein generating the embedded data set comprises generating for each data sample in the common data set an embedded sample, and
    wherein applying the embedded data set as an input to the predictor comprises applying only the embedded samples corresponding to the gene transcription data samples as an input to produce respective predicted values of the time-varying property for the embedded data samples corresponding to the data samples; and
    after obtaining the predictor, applying the embedded samples corresponding to the further data samples to the predictor to predict respective values of the time-varying property for the further cell samples.

12. The method of any preceding claim, wherein the number of dimensions of the embedded samples is selected based on the respective prediction performance of embedded data sets having different respective numbers of dimensions.

13. The method of any preceding claim, wherein the time-varying property is of one or more organisms or subjects from which the cell samples have been obtained, and further comprising generating a report that identifies a value of the time-varying property for the one or more organisms or subjects.

14. The method of any preceding claim, wherein the cell samples are single cell samples of a single cell each.

15. The method of any preceding claim, wherein the time-varying property is biological age, chronological age or a state of progression of a condition or disease, optionally wherein the condition or disease is a neurodegenerative disease or a cancer, optionally wherein the neurodegenerative disease is Alzheimer's disease or Parkinson's disease.

16. The method of any preceding claim, wherein the transcription levels and, where present, the further transcription levels have been derived from transcription counts of gene transcripts in the cell samples without use of knowledge of gene pathways.

17. The method of claim 16, comprising generating the embedded data set without use of knowledge of gene pathways and/or applying the embedded data set and obtaining the predictor without use of knowledge of gene pathways.

Figure 1

210 — Receive 1st gene transcription data set

Receive 2nd gene transcription data set — 212

Generate combined data set with samples from 1st+2nd sets — 214

220 — Generate combined embed-ded data set

230 — Train predictor from combined embedded data set

240 — Adjust prediction coefficients to fit predictor

Provide report — 250

Figure 2

310 — Receive 1st gene transcription data set

Receive 2nd gene transcription data set — 312

Generate combined data set with samples from 1st+2nd sets — 314

320 — Generate combined embedded data set

330 — Train predictor from combined embedded data set (samples of 1st set)

Predict property from combined embedded data set (samples of 2nd set) — 350

340 — Adjust prediction coefficients to fit predictor

Provide report — 360

Figure 3

Figure 4

Figure 5

Figure 6

Figure 6 (continued)

Figure 6 (continued)

G — B cells

Expr. clock

MAE (mo) = 5.94

Cor = 0.81

RD clock

MAE (mo) = 6.51

Cor = 0.78

Figure 6 (continued)

Figure 7

Figure 8

Europäisches
Patentamt

European
Patent Office

Office européen
des brevets

# EUROPEAN SEARCH REPORT

**Application Number**

EP 21 18 6218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEYER DAVID H. ET AL: "BiT age: A transcriptome-based aging clock near the theoretical limit of accuracy", AGING CELL, [Online] vol. 20, no. 3, 3 March 2021 (2021-03-03), XP055874427, GB ISSN: 1474-9718, DOI: 10.1111/acel.13320 Retrieved from the Internet: URL:https://onlinelibrary.wiley.com/doi/full-xml/10.1111/acel.13320> [retrieved on 2021-12-19] | 1-17 | INV. G16B25/10 G16B5/30 G16B40/20 |
| Y | * the whole document * * in particular * * sections 2 and 5 * | 1-17 | |
| X | FLEISCHER JASON G. ET AL: "Predicting age from the transcriptome of human dermal fibroblasts", GENOME BIOLOGY, [Online] vol. 19, no. 1, 20 December 2018 (2018-12-20), XP055874543, DOI: 10.1186/s13059-018-1599-6 Retrieved from the Internet: URL:https://genomebiology.biomedcentral.com/track/pdf/10.1186/s13059-018-1599-6.pdf> [retrieved on 2021-12-19] * the whole document * * in particular * * page 2 – page 5 * | 1-17 | |

**TECHNICAL FIELDS SEARCHED (IPC)**

G16B

-/--

The present search report has been drawn up for all claims

| Place of search | Date of completion of the search | Examiner |
|---|---|---|
| Munich | 20 December 2021 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 18 6218

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOLZSCHECK NICHOLAS ET AL: "Modeling transcriptomic age using knowledge-primed artificial neural networks", NPJ AGING AND MECHANISMS OF DISEASE, [Online] vol. 7, no. 1, 1 June 2021 (2021-06-01), XP055874546, DOI: 10.1038/s41514-021-00068-5 Retrieved from the Internet: URL:https://www.nature.com/articles/s41514-021-00068-5.pdf> [retrieved on 2021-12-19] * the whole document * * in particular * * "results and discussion" section; figure 1 * | 1-17 | |
| X | LOHMAN TREVOR ET AL: "Predictors of Biological Age: The Implications for Wellness and Aging Research", GERONTOLOGY AND GERIATRIC MEDICINE, [Online] vol. 7, 1 January 2021 (2021-01-01), XP055874561, ISSN: 2333-7214, DOI: 10.1177/23337214211046419 Retrieved from the Internet: URL:https://europepmc.org/backend/ptpmcrender.fcgi?accid=PMC8477681&blobtype=pdf> [retrieved on 2021-12-19] * the whole document * * in particular * * page 6; table 4 * | 1-17 | TECHNICAL FIELDS SEARCHED (IPC) |

-/--

The present search report has been drawn up for all claims

| Place of search | Date of completion of the search | Examiner |
|---|---|---|
| Munich | 20 December 2021 | Rákossy, Z |

## EUROPEAN SEARCH REPORT

Application Number

EP 21 18 6218

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | LALEH HAGHVERDI ET AL: "Batch effects in single-cell RNA-sequencing data are corrected by matching mutual nearest neighbors", NATURE BIOTECHNOLOGY, vol. 36, no. 5, 2 April 2018 (2018-04-02), pages 421-427, XP055593057, New York ISSN: 1087-0156, DOI: 10.1038/nbt.4091 * the whole document * * in particular * * "results" section; figure 1 * | 1-17 | |

TECHNICAL FIELDS
SEARCHED (IPC)

The present search report has been drawn up for all claims

| Place of search | Date of completion of the search | Examiner |
|---|---|---|
| Munich | 20 December 2021 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160222448 A1
- US 20190185938 A1
- US 10325673 B2

**Non-patent literature cited in the description**

- **HOLZSCHECK et al.** *npj Aging Mech Dis,* 2021, vol. 7, 15
- **LIBERZON, A et al.** The molecular signatures database hallmark gene set collection. *Cell Syst.,* 2015, vol. 1, 417-425
- **ZOU, H. ; HASTIE, T.** Regularization and variable selection via the elastic net. *Journal of the Royal Statistical Society: Series B (Statistical Methodology),* 2005, vol. 67 (2), 301-320, https://doi.org/10.1111/j.1467-9868.2005.00503.x
- **HAGHVERDI, L. ; LUN, A. T. L. ; MORGAN, M. D. ; MARIONI, J. C.** Batch effects in single-cell RNA-sequencing data are corrected by matching mutual nearest neighbors. *Nature Biotechnology,* 2018, vol. 36 (5), 421-427, https://doi.org/10.1038/nbt.4091
- The Tabula Muris Consortium. A single-cell transcriptomic atlas characterizes ageing tissues in the mouse. *Nature,* 2020, vol. 583, 590-595, https://doi.org/10.1038/s41586-020-2496-1